# EUROPEAN PATENT APPLICATION

(11) **EP 0 567 324 A1**
(43) Date of publication of application: **27.10.1993**
(21) Application number: 93303117.1
(22) Date of filing: 21.04.1993
(51) Int. Cl.: A61K 31/505

(54) **Medicaments for treating gar-transformylase-dependent tumors**

(30) Priority: 23.04.1992 US 872469
(71) Applicant: ELI LILLY AND COMPANY, Indianapolis, Indiana 46285 (US); Sloan-Kettering Institute For Cancer Research, New York, New York 10021 (US)
(72) Inventor: Grindley, Gerald Burr, Indianapolis, Indiana 46250 (US); Young, Charles William, New York, New York 10021 (US)
(74) Representative: Tapping, Kenneth George

(57) **Abstract**

Methods of treating glycinamide ribonucleotide transformylase-dependent tumors with glycinamide ribonucleotide transformylase inhibitors and subsequent radiotherapy are provided. Also provided are methods of treating glycinamide ribonucleotide transformylase-dependent tumors with a folate binding protein binding agent, glycinamide ribonucleotide transformylase inhibitors and radiotherapy.

## Description

The present invention relates to novel methods of treating glycinamide ribonucleotide (GAR)-transformylase-dependent tumors in mammals.

GAR-transformylase inhibitors are antineoplastic agents which inhibit de novo purine biosynthesis in solid tumor cells in mammals (Pizzorro, e_t a_L, Cancer Res., 51:2291-2295 (1991). Exemplary antineoplastic agents known to have activity against such GAR-transformylase-dependent tumors include 5,10-dideaza-5,6,7,8-tet- rahydrofolic acid (DDATHF, also known as lometrexol; U.S. Pat. No. 4,684,653), and various derivatives (U.S. Pat. Nos. 4,845,216; 4,882,334, and 4,902,796). Other exemplary GAR-transformylase inhibitors include homofolates and derivatives thereof (U.S. Pat. No. 4,946,846; and Divekar, et al., Mol. Pharmacol., 11:319-325 (1975)), and 5,11-methenyltetrahydrohomofolate (Slieker, et al., Mol. Pharmacol., 25:294-302 (1984). However, there exists a need for improved therapeutic treatment of mammals having such dependent tumors.

There also exists a need to improve the efficacy of radiotherapy for treatment of tumors in mammals. A major cause for tumor radioresistance is the presence of intratumoral hypoxic cells, those cells which are oxygen deficient (Moulder, et aₗ., Cancer and Metastasis Rev., 5: 313-341 (1987). Various methods for improving radiotherapy via reduction or reversal of hypoxia have been attempted. Such methods include DNA-sensitization with compounds such as 2-nitroimidazoles, but use of these compounds may result in dose-limiting neu- rotoxicity. Alteration in oxygen delivery to tissues including altering the affinity of hemoglobin for oxygen, increasing the oxygen-carrying capacity of blood by the use of transfusion, or administering perfluorochemicals and hyperbaric oxygen have also been studied. This type of alteration in oxygen delivery has frequently caused rapid adaptation of the tumor to the new oxygen level and the sensitivity of the tumor following oxygenation has been observed to be identical to that in the anemic state. Furthermore, attempts to reduce or eliminate hypoxic cells in tumors by introducing agents which are toxic to hypoxic cells have met with limited success. See, e.g., Coleman, C.N., J. Natl. Cancer Inst., 80:310-317 (1988). Therefore, tumor cell hypoxia continues to be considered as a major cause of tumor cell resistance to radiotherapy.

This invention provides a method of treating GAR-transformylase-dependent tumors in mammals comprising administering a GAR-transformylase inhibitor or a pharmaceutically acceptable salt thereof, in a dose or doses sufficient to reduce intracellular purine ribonucleotide pools in tumor cells and exposing said tumors to ionizing radiation wherein said exposure is less than the exposure recommended in the art.

This invention further provides a method of treating GAR-transformylase-dependent tumors in mammals comprising treating said mammal with an amount of a folate protein binding agent selected from folic acid, (6R)-5-methyl-5,6,7,8-tetrahydrofolic acid, and (6R)-5-formyl-5,6,7,8-tetrahydrofolic acid, or a pharmaceutically acceptable salt thereof, sufficient to substantially block the folate binding protein, administering a GAR-transformylase inhibitor or a pharmaceutically acceptable salt thereof, in a dose or doses sufficient to reduce intracellular purine ribonucleotide pools in tumor cells; and exposing said tumor to ionizing radiation wherein said exposure is less than the exposure recommended in the art.

The invention provides a method of treating GAR-transformylase-dependent tumors in mammals by administering GAR-transformylase inhibitor to said mammal and exposing said tumor to ionizing radiation at less than that exposure recommended in the art.

The invention not only provides a novel method for the treatment of GAR-transformylase-dependent tumors in mammals, but may also provide a substantial reduction of radiation side-effects. Depending upon the morphological location of the tumor(s) to be treated, the stage of development of the tumor and the condition of the mammal to be treated, irradiation side effects which may be minimized or eliminated include, for example, hair loss, xerostomia, skin reaction, nausea, fatigue, radiation pneumonitis, hypothyroidism, sterility, pulmonary fibrosis, cardiac damage, growth abnormalities and second malignancies.

GAR-transformylase inhibitors are those compounds which effectively inhibit the biological activity of the enzyme known as glycinamide ribonucleotide transformylase. This enzyme, the first folate-dependent enzyme of de novo purine synthesis in mammals, is implicated in DNAsynthesis. Thus, interruption of this biosynthetic pathway, especially in tumor cells which depend upon GAR-transformylase for growth, causes a disturbance of macromolecular synthesis (DNA, RNA and proteins), and consequently may cause cell inhibition or death. Any compound which is shown to inhibit GAR-transformylase is included within the scope of this invention.

More specifically, there is an absolute requirement for net purine synthesis in order to supply precursors for the new DNA and RNA synthesis necessary for cell sustinence, growth and division. In the absence of net purine synthesis via inhibition of the GAR-transformylase enzyme and the absence of a sufficient supply of purines via salvage pathways, intracellular purine ribonucleotide pools are depleted, presumably as a result of attempted DNA and RNA synthesis as well as ongoing irreversible oxidative purine catabolism.

Because there is a direct correlation between a GAR-transformylase inhibitor-induced reduction in intracellular purine ribonucleotide pools in tumor cells and observable GAR-transformylase inhibitor-induced cytotoxicity of tumor cells, an uninvasive observation of the latter is a positive indicator of the former. However, when a physician determines that a more accurate determination of the level of intracellular purine ribonucleotide pools is necessary, a tumor tissue biopsy may be performed and such ribonucleotide pool levels may be determined via methods known in the art. Such a biopsy may be performed as soon as about 4 to about 12 hours after the administration of a GAR-transformylase inhibitor, regardless as to whether such administration is a single dose or one of the doses in a multidose course of treatment.

One method for determining the level of intracellular purine ribonucleotide pools is described by Pizzorno, et al., Cancer Res. 51:2291-2295 (1991). Cells (5-10 x 10⁶) are collected and extracted with 1 M formic acid saturated with n-butyl alcohol on ice. The extract is lyophilized and reconstituted with mobile phase before high performance liquid chromatography analysis. Separation of nucleotides is performed on a Whatman Partisil-10-SAX anion exchange column (Whatman, Woburn, MA.), using a 0.4 M ammonium phosphate isocratic elution at a flow rate of 1.5 mL/min. Eluted nucleotide triphosphates are monitored with a Model 153 Altex detector set (Berkeley, CA.), set at 254 nm. The retention peak areas are determined with a Shimadza C-RIA Chroma- topue Integrator (Kyoto, Japan).

Treatment of mammalian tumor cells with a GAR-transformylase inhibitor, particularly DDATHF, is known to selectively induce such a depletion of intracellular ribonucleotide pools. Tumoric cell death is frequently a consequence of this depletion. However, those cells which do not die are usually left in a de-energized state which results in a reduction of phosphorylation of nucleosides to activated DNA and RNA precursors, and a diminished rate of macromolecular synthesis overall. It appears as if the reduction in nucleoside phosphorylation would further result in the conservation of intracellular oxygen. This factor becomes more important in the discussion below.

Typical GAR-transformylase inhibitors include the pyrido[2,3-d]pyrimidine derivatives described by Taylor, et al. in U.S. Pat. Nos. 4,684,653, 4,845,216, and 4,882,334. Another series of GAR-transformylase inhibitors has been described by Nomura, et al. in U.S. Pat. No. 4,946,846. All of the foregoing references are incorporated herein by reference for their teaching of the structure and synthesis of typical GAR-transformylase inhibitors. Other GAR-transformylase inhibitors are also included within the scope of this invention and such compounds can be determined by routine evaluation of their ability to interact with and inhibit the subject enzyme.

A preferred GAR-transformylase inhibitor of the invention is a 5,10-dideazafoiic acid derivative of the formula 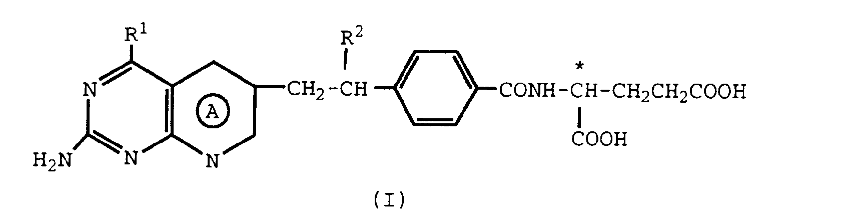
wherein
A is pyrido or tetrahydropyrido;
R¹ is amino or hydroxy;
R² is hydrogen, methyl or ethyl;
the configuration about the carbon atom designated ^{*} being L;

or a pharmaceutically-acceptable salt thereof.

An especially preferred compound is 5,10-dideaza-5,6,7,8-tetrahydrofoiic acid or a pharmaceutically-acceptable salt thereof.

As mentioned above, the invention includes the use of pharmaceutically acceptable salts of GAR-transformylase inhibitors and folate binding protein binding agents. A particular GAR-transformylase inhibitor orfo- late binding protein binding agent can possess a sufficiently acidic, a sufficiently basic, or both functional groups, and accordingly react with any of a number of nontoxic inorganic bases, and nontoxic inorganic and organic acids, to form a pharmaceutically acceptable salt. Acids commonly employed to form acid addition salts are inorganic acids such as hydrochloric acid, hydrobromic acid, hydroiodic acid, sulfuric acid, phosphoric acid, and the like, and organic acids such as p-toluene-sulfonic, methanesulfonic acid, oxalic acid, p-bromo-phenylsulfonic acid, carbonic acid, succinic acid, citric acid, benzoic acid, acetic acid, and the like. Examples of such pharmaceutically acceptable salts thus are the sulfate, pyrosulfate, bisulfate, sulfite, bisulfite, phosphate, monohydrogenphosphate, dihydrogenphosphate, metaphosphate, pyrophosphate, chloride, bromide, iodide, acetate, propionate, decanoate, caprylate, acrylate, formate, isobutyrate, caproate, heptanoate, propiolate, oxalate, malonate, succinate, suberate, sebacate, fumarate, maleate, butyne-1,4- dioate, hexyne-1,6-dioate, benzoate, chlorobenzoate, methylbenzoate, dinitrobenzoate, hydroxybenzoate, methoxybenzoate, phthalate, sulfonate, xylenesulfonate, phenylacetate, phenylpropionate, phenylbutyrate, citrate, lactate, gamma-hydroxybutyrate, glycollate, tartrate, methanesulfonate, propanesulfonate, naphthalene-1-sulfonate, naphthalene-2-sulfonate, mandelate,and the like. Preferred pharmaceutically acceptable acid addition salts are those formed with mineral acids such as hydrochloric acid and hydrobromic acid, and those formed with organic acids such as maleic acid and methanesulfonic acid.

Base addition salts include those derived from nontoxic inorganic bases, such as ammonium or alkali or alkaline earth metal hydroxides, carbonates, bicarbonates, and the like. Such bases useful in preparing the salts of this invention thus include sodium hydroxide, potassium hydroxide, ammonium hydroxide, potassium carbonate. The potassium and sodium salt forms are particularly preferred.

A GAR-transformylase inhibitor dosage and method of administration which is sufficient to reduce intracellular purine ribonucleotide pools and tumor cells is equivalent to those dosages taught in the art for the treatment of GAR-transformylase inhibitor-susceptible neoplasms or GAR-transformylase-dependent neoplastic (tumor) growth. Exemplary susceptible neoplasms or neoplastic growth include but are not limited to chlorio- carcinoma, leukemia, adenocarcinoma of the female breast, carcinoma of the ovary, epidermoid cancers of the head and neck, squamous or small-cell lung cancer, and various lymphosarcomas. See, e.g., U.S. Pat. Nos. 4,684,653 and 4,845,216.

Typically, GAR-transformylase inhibitors can be administered to mammals alone or in combination with other therapeutic agents including other antineoplastic agents, steroids and the like, and may be administered as such or they can be compounded and formulated into pharmaceutical compositions in unit dosage form for parenteral and oral administration. Such pharmaceutical compositions are prepared in a manner well known in the art and comprise at least one GAR-transformylase inhibitor associated with a pharmaceutically acceptable carrier.

In such a composition, the active compound and, if included, other therapeutic agents, are known as active ingredients. In making the compositions, the active ingredient(s) will usually be mixed with a carrier, or diluted by a carrier, or enclosed within a carrier which may be in the form of a capsule, sachet, paper or other container. When the carrier serves as a diluent, it may be a solid, semisolid or liquid material which acts as a vehicle, excipient or medium for the active ingredient. Thus, the composition can be in the form of tablets, pills, powders, lozenges, sachets, cachets, elixirs, emulsions, solutions, syrups, suspensions, soft and hard gelatin capsules, sterile injectable solutions, and sterile packaged powders. Some examples of suitable carriers, excipients, and diluents include lactose, dextrose, sucrose, sorbitol, mannitol, starches, gum acacia, calcium phosphate alginates, calcium silicate, microcrystalline cellulose, polyvinylpyrrolidone, cellulose derivatives, tragacanth, gelatin, syrup, methyland propyl-hydroxybenzoates, talc, magnesium stearate, water, and mineral oil. The formulations can additionally include lubricating agents, wetting agents, emulsifying and suspending agents, preserving agents, sweetening agents or flavoring agents. The compositions may be formulated so as to provide quick, sustained, or delayed release of the active ingredient after administration to the patient by employing procedures well known in the art. For oral administration, a GAR-transformylase inhibitor, optionally including other therapeutic agents, can be admixed with carriers and diluents molded into tablets or enclosed in gelatin capsules. The mixtures can alternatively be dissolved in liquids such as 10% aqueous glucose solution, isotonic saline, sterile water, or the like, and administered via parenteral routes including intramuscular, intrathecal, intravenous and intra-arterial. Such solutions will contain-from about 0.5% to about 50% by weight of a GAR-transformylase inhibitor, ideally about 1% to about 20%.

The compositions are preferably formulated in a unit dosage form, each dosage containing from about 1 to about 500 mg and, more frequently, from about 5 to about 300 mg of the active ingredient(s). The term "unit dosage form" refers to physically discrete units suitable as unitary dosages for human subjects and other mammals, each unit containing a predetermined quantity of active material calculated to produce the desired therapeutic effect, in association with the required pharmaceutical carrier. Such compositions may contain a GAR-transformylase inhibitor as an active ingredient or may contain a GAR-transformylase inhibitor plus another therapeutic agent as active ingredients.

The active GAR-transformylase inhibitors are effective over a wide dosage range. For example, daily dosages will be found to be within the range of about 0.1 mg/M² to about 500 mg/M² of body weight. In the treatment of adult humans, the dosage range from about 1 mg/M² to about 300 mg/M², in single or divided doses, is preferred. Ideal dosages range from about 10 _{M}g/M² to about 250 mg/M². However, it will be understood that the amount of the compound actually administered will be determined by a physician in light of the relevant circumstances including the relative severity of the tumor, the choice of compound or compounds to be administered, the age, weight, and response of the individual patient, and the chosen route of administration. Therefore, the above dosage ranges are not intended to limit the scope of this invention in any way. Dosage ranges for other therapeutic agents should be used according to recommendations for each agent.

As mentioned above, the efficacy of radiotherapy for treatment of tumors in mammals is frequently ineffective because of tumoral cell radioresistence via the presence of hypoxic cells. The presence of hypoxic cells and the means by which hypoxia protects the cells from the lethal effects of ionizing radiation has been known since the 1930's.

In essence, exposure of tumors to ionizing radiation produces free radical injury to DNA. The extent of the cellular injury produced at a constant radiation dosage is influenced by the magnitude of the original acute injury to cellular DNA, by the rate and precision with which the affected cells repair the treatment-induced injury, and by the cells' metabolic and proliferative state. An enhanced therapeutic effect could be achieved if the magnitude of the initial DNA damage were increased and the rate of DNA repair were slowed. The extent of acute DNA injury is greatly influenced by tissue levels of oxygen since the free radicals react with oxygen to form a peroxyl or hydroxyl radical which subsequently forms products that are different from the original target molecules. This type of damage requires extensive enzymatic action to restore the DNA to its pre-injury form. These steps include addition of poly(ADP-ribose) polymers to nuclear proteins at the site of injury, ex- cission of the injured area and adjoining segments of DNA and, finally, repair synthesis of the DNA. Alternatively, in the absence of sufficient oxygen, the DNA radicals can react with reducing species, such as the thiols (-SH), which can donate hydrogen and restore the target molecule to its original form.

Repair of radiation injury to DNA requires normal function of the cellular pyridine nucleotide cycle. This cycle is triggered by damaged DNA, and the level of the cycle's activity is proportional to the DNA damage incurred. Upon cycle activation, poly(ADP-ribose) polymerase catalyzes the successive transfer of ADP-ribosyl groups from nicotinamide adenine dinucleotide (NAD) to nuclear proteins. As the cycle progresses, NAD is consumed and regeneration of NAD from nicotinamide, via a series of ATP-requiring reactions, is required if poly(ADP-ribose) formation is to continue. When DNA injury is severe, cellular content of NAD, and thereafter ATP, becomes depleted. Because administration of a GAR-transformylase inhibitor selectively lowers tumor cell content of intracellular purine ribonucleotide pools, leaves the cells in a de-energized state, and presumably conserves available cellular oxygen via the resultant reduction or elimination of nucleoside phosphorylation, exposure to ionizing radiation would enhance damage to tumoral DNA, accelerate NAD consumption in cells which have a GAR-transformylase-induced limitation on regeneration of NAD from nicotinamide, and further reduce the cells' ability to recover from a de-energized state. Thus, the effect from administration of a GAR-transformylase inhibitor plus the irradiation of a target tumor results in the inhibition of tumor cell division and an increased rate of interphase cell death. This effect is frequently (i) greater than that observed following the administration of a GAR-transformylase inhibitor without radiotherapy; (ii) greater than that observed following radiotherapy without the administration of a GAR-transformylase inhibitor; and (iii) greater than the expected sum of a combination of the two treatments.

Specific radiation dosage recommendations are based upon a multiplicity of factors including, for example, the type, severity and location of the tumor(s), the condition of the patient, treatment history, known radiore- sistance/radiosensitivity of the tumor type, tissue tolerances and tumor control probabilities. Recommendations will usually assist in selecting beam directions and shapes, the type of radiotherapy (e.g., X-rays , gamma- rays, particle streams, etc.), the number and frequency of each fraction, and especially, the exposure per fraction and total exposure. See, e.g., Important Advances in Oncology 1991 (Vincent T. DeVita, Jr., et al. eds., 1991); Cecil Textbook of Medicine (James B. Wyngaarden, et al. eds., 18th ed. 1988); Conn's Current Therapy 1990 (Robert E. Rakel ed., 1990).

The methods of the invention permit a physician to substantially reduce the total radiation exposure compared to the exposure recommended in the art. Total radiation exposure is the sum of individual fractional exposures. Typically, the total radiation exposure may be reduced by about 10 to about 60 percent of the total radiation exposure recommended in the art, while still maintaining a therapeutically acceptable amount of ionizing radiation. Preferably, total radiation exposure will be reduced by about 20 to about 30 percent of such recommendations.

For each tumoral condition, the recommended number and frequency of radiation fractions is also known in the art. Physicians should follow those recommendations. When such recommendations allow the physician to select a radiotherapy course of treatment from a range of recommendations, a greater number of fractions, administered at lower dosages is preferred. Such fractionation at lower total radiation exposures provides an improved therapeutic index while improving tissue tolerances and lessening the impact of acute and subacute side effects.

Radiotherapy should commence from about4 hours following administration of a single dose, orfirst GAR-transformylase inhibitor dose given in a multidose course of treatment, to about 60 days following the single or final administration in a multidose course of treatment. Commencement of radiotherapy from about 24 hours to about 48 hours after beginning administration of such a single or multidose course of treatment is preferred.

The invention further provides a method of treating GAR-transformylase-dependent tumors in mammals comprising treating said mammal with an amount of a folate binding protein binding agent selected from folic acid, (6R)-5-methyl-5,6,7,8-tetrahydrofolic acid, and (6R)-5-formyl-5,6,7,8-tetrahydrofolic acid, or physiologically-available salt or ester thereof, sufficient to substantially block the folate binding protein; administering a GAR-transformylase inhibitor in a dose or doses sufficient to reduce intracellular purine ribonucleotide pools in tumor cells; and exposing said tumor to ionizing radiation wherein said exposure is less than the exposure recommended in the art.

Folate binding protein (FBP) binding agents are compounds such as folic acid, (6R)-5-methyl-5,6,7,8-tet- rahydrofolic acid and (6R)-5-formyl-5,6,7,8-tetrahydrofolic acid. These compounds, in addition to other antifolate agents which bind to FBP (see, e.g., Kane et al., Laboratory Investigation, 60:737(1989)), significantly reduce the toxic effects of GAR-transformylase inhibitors without adversely affecting therapeutic efficacy. See, e.g., Grindey, et al., Proceedings of the 82nd Annual Meeting of the American Association for Cancer Research, Vol. 32, pg. 384, Abst. 1921 (1991).

Folic acid is a vitamin which is required by mammals for proper regeneration of the blood-forming elements and theirfunctioning, and as a coenzyme is involved in intermediary metabolic processes in which one-carbon units are transferred. These reactions are important in interconversions of various amino acids and in purine and pyrimidine synthesis. Folic acid is commonly supplied to diets of humans via consumption of food sources such as liver, kidney, dry beans, asparagus, mushrooms, broccoli, lettuce, milk and spinach, as well as by vitamin supplements. The minimum amount of folic acid commonly required by normal adults is about 0.05 mg/day. According to this invention, folic acid, or a physiologically-available salt or ester thereof, is administered to a human subject at a dose of about 0.5 mg/day to about 30 mg/day to diminish the toxic effects of a GAR-transformylase inhibitor also being administered to such subject. In a preferred embodiment, folic acid will be administered at about 1 to about 5 mg/day together with the normal dosing of a GAR-transformylase inhibitor such as lometrexol.

(6R)-5-formyi-5,6,7,8-tetrahydrofoiicacid is the (6R) isomer of leucovorin. Leucovorin is disclosed in J. Am. Chem. Soc., 74:4215 (1952). This compound and (6R)-5-methyl-5,6,7,8-tetrahydrofolic acid are in the unnatural configuration at the 6-position, and are 10-20 fold more efficient in binding the folate binding protein compared with their respective (6S)-isomer (see, Ratnam et aₗ., Folate and Antifolate Transport in Mammalian Cells Symposium, March 21-22, 1991, Bethesda, Maryland). These compounds are usually prepared as a mixture with their natural form (6S) of diastereomers by non-stereoselective reduction from the corresponding dehydro precursors followed by separation through chromatographic or enzymatic techniques. See e.g., PCT Patent Application Publication WO 8808844 (also Derwent Abstract 88-368464/51) and Canadian Patent 1,093,554.

Based upon the relative binding constants for the respective compounds, it will be expected that approximately 1 mg/day to 90 mg/day (preferably approximately 2-15 mg/day) of (6R)-5-methyl-5,6,7,8-tetrahydrofolic acid or about 5-300 mg/day (preferably about 10-5- mg/day) of (6R)-5-formyl-5,6,7,8-tetrahydrofolic acid, or their respective pharmaceutically acceptable salt thereof, will be employed with the GAR-transformylase inhibitor.

The FBP binding agent to be utilized according to this invention can be in its free acid form, or can be in the form of a pharmaceutically acceptable salt. The dosage generally will be provided in the form of a vitamin supplement, namely as a tablet administered orally, preferably as a sustained release formulation, as an aqueous solution added to drinking water, an aqueous parenteral formulation, e.g., an intravenous formulation or the like.

The FBP binding agent may be administered to the subject mammal prior to the administration of a GAR-transformylase inhibitor, concomitantly with administration of a GAR-transformylase inhibitor, or both. Pretreatment with the suitable amount of FBP binding agent from about 1 to about 24 hours is preferred and is usually sufficient to substantially bind to and block the folate binding protein prior to administration of the GAR-transformylase inhibitor. Although one single dose of the FBP binding agent, preferably an oral administration of folic acid, should be sufficient to load the folate binding protein, multiple dosing of the FBP binding agent can be employed for periods up to weeks before treatment with the GAR-transformylase inhibitor to ensure that the folate binding protein is sufficiently bound in order to maximize the benefit derived from such pretreatment. In addition, administration of a FBP binding agent may continue throughout a multidose course of treatment with GAR-transformylase inhibitors.

In the especially preferred embodiment of this invention, about 1 mg to about 5 mg of folic acid is administered orally to a mammal about 1 to about 24 hours prior to the parenteral administration of the amount of lometrexol which is normally required to attain the desired therapeutic benefit. Although greater or additional doses of folic acid or another FBP binding agent are also operable, the above parameters will usually bind the folate binding protein in an amount sufficient to reduce the toxicity effects normally seen upon lometrexol administration as described above.

It should be noted that the FBP binding agent is not an antitumor agent and that the treatment of a mammal with FBP binding agent is not a synergistic or potentiating effect. Rather, by having substantially bound the folate binding protein with a FBP binding agent, the toxic effects of GAR-transformylase inhibitor treatments are greatly reduced without affecting the therapeutic efficacy.

The following specific examples are shown to further assist the reader in using the methods of the present invention. However, these specific examples are not intended to be limiting on the scope of the invention

### Example 1

A 36 year old man was ill with an aggressive soft tissue sarcoma characterized as either a malignant Schwannoma or a tendosynovial sarcoma. At the time of entry into therapy with lometrexol, the disease was present in his lungs, mediastinum and the chest wall. Prior therapy included resection of the primary liaison and of multiple pulmonary metastases. The patient had also experienced disease progression despite the following chemotherapeutic medications: ifosfamide, dacarbazine, adriamycin, PALAand thioTEPA. Lometrexol was administered at a dosage of 4.8 mg/m² (10 mg total) on the following dates 5/4/90, 5/7/90, 5/11/90, and 5/14/90. The patient developed mild mucositis due to the drug, and subsequent thrombocytopenia to 55,000. The latter condition required leucovorin administration and hospital admission on 5/30/90.

Regardless of the treatment, the tumor continued to rapidly progress and by 6/6/90, the patient had developed marked facial edema, tracheal compression, and the tumor mass involved most of the right atrium of the heart with pulmonary hypertension. Although sarcomatous tumors of this nature are usually radioresistant and require radiation dosages in excess of 5,000 centiGray (cGy) to produce any demonstrable effect, the patient was begun on palliative radiation on an emergency basis. At the time the radiation treatment was initiated, his projected life expectancy was a matter of days. He received 2,000 cGy in 4 fractions of 500 cGy each to his neck and mediastinum, using a 15 Mev linear excelerator unit. Treatment was given on 6/7/90, 6/8/90, 6/11/90, and 6/12/90. The clinical response in the irradiated field was dramatic, with rapid disappearance of signs and symptoms of the superior caval obstruction and disappearances of the palpable tumor mass on the chest wall.

Although the disease within the irradiated field was controlled, it was believed that irradiation of all of the patient's known disease was not feasible. Accordingly, the patient was discharged to home hospice care on 6/19/90, and eventually died of his disease on 8/22/90.

At the time radiotherapy was initiated, it was evident that lometrexol retention in the patients body was prolonged based on the observation that the patient was still thrombocytopenic from lometrexol.

### Example 2

A 31 year old woman was ill with an adenocarcinoma originating in the ethmoid sinus. At the time the patient was started on lometrexol, the disease was metastatic in the supraclavicular area, the retrosternal area and the liver. Prior therapy in May and June of 1988 had included control of the local primary liaison by combined cisplatin chemotherapy and hyperfractionated external beam radiation therapy at 5400 cGy directed to the paranasal sinuses and nasopharynx, and 7000 cGy to the primary site of the tumor. Tumor metastases were present in December, 1989, and the disease progressed in spite of therapy with cisplatin plus 5-fluoro- uracil, cyclophosphamide, adriamycin, and cisplatin alone. Lometrexol was then administered on 9/28/90, 10/1/90, 10/5/90 and 10/8/90 at a dosage of 6.4 mg/m²/dose (10.4 mg total) combined with folic acid at 1 mg/day. This regimen produced prolonged thrombocytopenia and anemia for over 7 weeks. At this time the patient's platelet count began to recover but her liver was growing rapidly in size. Whole liver radiation therapy was begun on 12/11/90 and the patient was hospitalized on 12/12/90. Although radiation therapy was initiated about 2 months following the final administration of lometrexol, persistent anemia at a Hgb of 5.4 demonstrated a continuing lometrexol effect. The patient received 2100 cGy in 7 fractions of 300 cGy from a 6 Mev linear accelerator unit. Therapy was given between 12/11/90 and 12/21/90. Following this method of treatment, the patient experienced marked shrinkage of her massively enlarged liver. The patient also developed ascites during the initial phase of her admission to the hospital. However, the ascites were coming under control at the time of discharge. The patient eventually died of her disease on March 6, 1991.

It is not common oncologic practice to irradiate hepatic metastases because the objective response rate is negligible. This patient was referred for whole liver irradiation because she was acutely uncomfortable from stretching of the hepatic capsule, and because the patient described in Example 1 had experienced an unexpectedly favorable response to irradiation therapy administered subsequent to lometrexol treatment. The magnitude of tumor regression observed in this patient is highly unexpected in view of the low total dosage administered and the poor global response rate usually obtained with irradiation of hepatic metastases.

## Claims

1. The use of a GAR-transformylase inhibitor or a pharmaceutically acceptable salt thereof, in the preparation of a medicament useful for treating a patient afflicted with cancer receiving or about to receive adjunct ionizing radiation therapy.

2. The use as claimed in Claim 1 wherein the GAR-transformylase inhibitor is a 5,10-dideazafolic acid of the formula 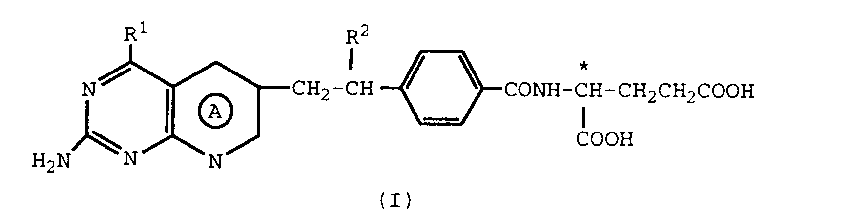
wherein
A is pyrido or tetrahydropyrido;
R¹ is amino or hydroxy;
R² is hydrogen, methyl or ethyl:
the configuration about the carbon atom designated ^{*} being L;
or a pharmaceutically acceptable salt thereof.

3. The use as claimed in Claim 2 wherein the GAR-transformylase inhibitor is 5,10-dideaza-5,6,7,8-tetrahy- drofolic acid or a pharmaceutically acceptable salt thereof.

4. The use of a GAR-transformylase inhibitor or pharmaceutically acceptable salt thereof, and a folate binding protein binding agent selected from folic acid, (6R)-5-methyl-5,6,7,8-tetrahydrofolic acid, and (6R)-5- formyl-5,6,7,8-tetrahydrofolic acid or a physiologically-available salt or ester thereof, in the preparation of a medicament useful for treating a patient afflicted with cancer receiving or about to receive adjunct ionizing radiation therapy.

5. The use as claimed in Claim 4 wherein the GAR-transformylase inhibitor is a 5,10-dideazafolic acid of the formula 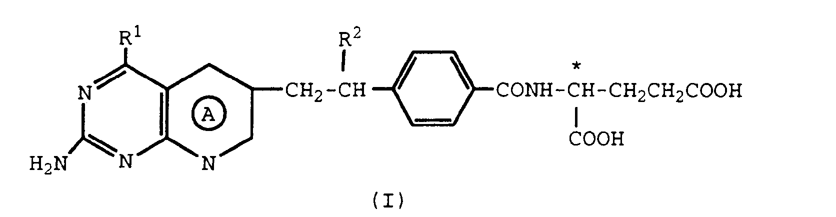
wherein
A is pyrido or tetrahydropyrido;
R¹ is amino or hydroxy;
R² is hydrogen, methyl or ethyl:
the configuration about the carbon atom designated ^{*} being L;
or a pharmaceutically acceptable salt thereof.

6. The use as claimed in Claim 5 wherein the GAR-transformylase inhibitor is 5,10-dideaza-5,6,7,8-tetrahy- drofolic acid or a pharmaceutically acceptable salt thereof.

7. The use as claimed in Claim 4 wherein the folate binding protein binding agent is folic acid or a physiologically-available salt or ester thereof.

8. The use as claimed in Claim 7 wherein the GAR-transformylase inhibitor is 5,10-dideaza-5,6,7,8-tetrahy- drofolic acid or a pharmaceutically acceptable salt thereof.

9. The use of a GAR-transformylase inhibitor or a pharmaceutically acceptable salt thereof in the treatment of a patient suffering from cancer and receiving or about to receive adjunct ionizing radiation therapy.

10. A combination of a GAR-transformylase inhibitor and ionizing radiation.

11. A combination of a GAR-transformylase inhibitor, a folate binding protein binding agent selected from folic acid, (6R)-5-methyl-5,6,7,8-tetrahydrofolic acid, and (6R)-5-formyl-5,6,7,8-tetrahydrofolic acid, and ionizing radiation.
